# EUROPÄISCHE PATENTSCHRIFT

(11) **EP 2 931 182 B1**
(45) Veröffentlichungstag und Bekanntmachung des Hinweises auf die Patenterteilung: **11.07.2018**
(21) Anmeldenummer: 13811868.2
(22) Anmeldetag: 16.12.2013
(51) Int. Cl.: A61F 2/44, A61F 2/46, A61F 2/28, A61F 2/30

(54) **LATERAL EXPANDIERBARES ZWISCHENWIRBELFUSIONSIMPLANTAT**
LATERALLY EXPANDABLE VERTEBRAL FUSION IMPLANT
IMPLANT DE FUSION INTERVERTÉBRAL EXPANSIBLE SUR LES CÔTÉS

(30) Priorität: 14.12.2012 DE 202012011960 U; 17.12.2012 EP 12197580
(43) Veröffentlichungstag der Anmeldung: 21.10.2015
(73) Patentinhaber: FACET-LINK Inc., Rockaway NJ 07866 (US)
(72) Erfinder: DMUSCHEWSKY, Klaus, 22397 Hamburg (DE)
(74) Vertreter: Glawe, Delfs, Moll
(86) Internationale Anmeldenummer: PCT/EP2013/076688
(87) Internationale Veröffentlichungsnummer: WO 2014/091029

(56) Entgegenhaltungen:
- WO-A1-00/25706
- WO-A1-2010/105181
- WO-A1-2012/115631
- WO-A2-2007/121320
- US-A1- 2008 243 255
- US-A1- 2009 157 084
- US-A1- 2011 276 141
- US-A1- 2012 123 546
- US-A1- 2012 197 299
- US-A1- 2012 209 386
- US-A1- 2012 271 422

## Beschreibung

Die Erfindung betrifft ein Zwischenwirbelfusionsimplantat zur Fusion zweier benachbarter Wirbel umfassend einen verstellbaren Tragkörper, dessen Boden- und Deckfläche zur Anlage an den Wirbeln ausgebildet sind. Zwischenwirbelfusionsimplantate sind aus dem Stand der Technik, beispielsweise WO 2007/121320 A2, bekannt.

Durch Verschleiß oder krankhafte Veränderungen kommt es an der Wirbelsäule zu einer Degeneration von Bandscheiben. Soweit konservative Therapie mit Medikation und/oder Physiotherapie nicht greift, ist mitunter eine operative Behandlung indiziert. Dazu ist es bekannt, in den Wirbelzwischenraum mit der degenerierten Bandscheibe ein bewegliches oder unbewegliches Implantat einzuführen. Dieses übernimmt die Tragfunktion der degenerierten Bandscheibe und stellt insoweit wieder eine sichere Abstützung zwischen den benachbarten Wirbeln her. Unbewegliche Implantate werden auch als "Fusionsimplantat" bezeichnet.

Zur Implantation der Fusionsimplantate sind verschiedene Operationstechniken bekannt. Eine klassische Operationstechnik besteht in einem Zugang von ventral, um so die Gefahr einer Beschädigung des Rückenmarks in der Wirbelsäule zu vermeiden. Dieser Vorteil wird jedoch erkauft mit einem ausgesprochen langen Zugangsweg durch den Bauch- oder Brustraum des Patienten. Da es hierbei zu Komplikationen kommen kann, ist ein alternativer Zugangsweg etabliert worden, nämlich von dorsal. Dieser bietet zwar den Vorteil eines kurzen Wegs, jedoch besteht die Gefahr einer Kollision bzw. Verletzung des Rückenmarks. Um diese Gefahr klein zu halten, erfolgt die Operation üblicherweise im Weg minimal invasiver Chirurgie. Derartige Zugänge von unmittelbar dorsal bzw. mehr von der Seite sind als Operationstechnik PLIF (posterior lumbar intervertebral fusion) bzw. TLIF (transforaminelle interkorporelle lumbare Fusion) bekannt, bei der die Bandscheibe von posterior bzw. lateral exponiert wird. Wegen der kleinen Querschnitte beim Zugang mittels minimal invasiver Chirurgie ist hierbei die Größe der Fusionsimplantate naturgemäß stark beschränkt.

Für die Therapie mittels der PLIF- bzw. TLIF-Technik sind sehr kleine Fusionsimplantate bekannt. Sie bieten den Vorteil, dass sie durch die minimal invasive Chirurgie dank ihrer Kleinheit implantiert werden können. Ein inhärenter Nachteil ihrer Kleinheit liegt jedoch darin, dass die Stützfunktion aufgrund der geringen Abmessungen eingeschränkt und mitunter unzureichend ist. Eine größere Ausführung der Fusionsimplantate würde die Stützfunktion zwar verbessern, jedoch ist dies nicht praktikabel aufgrund der Beschränkung der minimal invasiven Chirurgie.

Die Erfindung hat sich zur Aufgabe gesetzt, ein Fusionsimplantat der eingangs genannten Art dahingehend zu verbessern, dass bei weiterhin kleinem Zugangsquerschnitt, wie er für die minimal invasive Chirurgie üblich ist, dennoch eine bessere Stützwirkung erzielen kann.

Bei einem Zwischenwirbelfusionsimplantat zur Fusion zweier benachbarter Wirbel umfassend einen verstellbaren Tragkörper, dessen Boden- und Deckfläche zur Anlage an Endplatten der benachbarten Wirbel ausgebildet sind, ist erfindungsgemäß ein um ein Gelenk lateral schwenkbarer Seitenausleger vorgesehen, dessen Boden und Deckel flächenmäßig ausgedehnt sind, und ist weiter eine Betätigungseinrichtung vorgesehen zum Ausschwenken des Seitenauslegers in eine vom Tragkörper abgespreizte Position (Wirkstellung).

Die Erfindung beruht auf dem Gedanken, ein Zwischenwirbelfusionsimplantat zu schaffen, welches in einer Montagestellung besonders kleine Abmessungen aufweist und, nach der Montage am vorgesehenen Implantationsort, so betätigt werden kann, dass es sich vergrößert und damit eine größere Stützfläche zur Abstützung in Wirbelzwischenraum bietet. Letzterer Zustand wird als Wirkstellung bezeichnet. Damit vereinigt das erfindungsgemäße Implantat den Vorteil des Zugangs durch eine verhältnismäßig kleine Zugangsöffnung, wie sie typisch ist für die minimal invasive Chirurgie, mit dem Vorteil einer verhältnismäßig großen Abstützungsfläche, wie sie für auf wesentlich invasivere Weise implantierte, konventionelle Implantate typisch ist.

Die Erfindung beruht auf dem Gedanken, durch einen seitlichen Schwenkmechanismus das Implantat zur Montage möglichst klein zu gestalten und am vorgesehenen Implantationsort durch Ausschwenken möglichst groß zu gestalten. Hierbei werden die gegensätzlich erscheinenden Ziele der Kleinheit zur Implantation einerseits und der möglichst großen Aufstützfläche für die Fusion der Wirbel miteinander verknüpft. Das erfindungsgemäße Implantat eignet sich damit auch zur Versorgung verhältnismäßig großflächiger Defekte einer Bandscheibe, und zwar auch dann, wenn die Operation lediglich im Wege der minimal invasiven Chirurgie vorgenommen werden soll.

Erfindungsgemäß ist der Seitenausleger als ein Kniehebelspreizer ausgeführt, der einen Schwenkarm mit einem Spreizarm umfasst. Damit ergibt sich eine etwa dreiecksförmige Konstruktion, welche eine gute und sichere Führung des ausschwenkenden Arms ermöglicht, und der Gefahr von Verkantungen beim Ausschwenken, welche zu einem Blockieren des Implantats führen könnten, wird damit wirkungsvoll begegnet. Erfindungsgemäß wird der Spreizarm in der Wirkstellung am Tragkörper verriegelt. Mit einer solchen Verriegelung wird sichergestellt, dass auch bei dem Auftreten großer Lasten die Wirkstellung beibehalten wird. Erfindungsgemäß ist an dem Spreizarm ein Gleitstück befestigt, welches von der Betätigungseinrichtung beim Spreizen verschoben, insbesondere zurückgezogen wird und bei Erreichen der Wirkstellung vorzugsweise in Ausnehmungen verrastet. Durch die Verrastung erfolgt eine formschlüssige Verriegelung, welche eine besonders hohe Sicherheit gegenüber einem unerwünschten Herausbewegen bietet.

Zur Gewährleistung einer möglichst geringen Abmessung des erfindungsgemäßen Implantats bei der Montage ist vorzugsweise der Seitenausleger in den Tragkörper eingezogen. Mit einer solchen Positionierung wird die kleinstmögliche Gestaltung erreicht. Auf diese Weise kann der Tragkörper maximal groß gewählt werden, dementsprechend kann auch der darin eingezogene Seitenausleger recht groß gewählt werden.

Um das Einführen zu erleichtern und der Gefahr eines Verhakens an umliegendem Gewebe entgegenzuwirken, sind der Boden und der Deckel des Seitenauslegers vorzugsweise so gestaltet, dass sie in der Montagestellung bündig an den Boden- bzw. Deckflächen des Tragkörpers anliegen. Damit ergibt sich eine durchgehende Fläche ohne Zwischenraum. Besonders bewährt ist es, wenn der Boden und der Deckel des Seitenauslegers in einer Ebene liegen, d. h. sie fluchten mit der Boden- und/oder Deckfläche des Tragkörpers. Damit wird erreicht, dass der Seitenausleger nach dem Aufspreizen mit seinem Boden und seinem Deckel zuverlässig Kontakt mit den Endplatten der beiden benachbarten Wirbeln hat, und zwar in derselben Weise wie der Tragkörper selbst.

Um ein Einwachsen des erfindungsgemäßen Implantats im Wirbelzwischenraum zu begünstigen, kann es vorgesehen sein, dass an der Boden- und/oder Deckfläche des Tragkörpers und/oder am Boden oder Deckel des Seitenauslegers Schneidzähne angeordnet sind. Mit den Schneidzähnen kann insbesondere bei der Spreizbewegung eine Anfrischung der Endplatte erreicht werden, wodurch das natürliche Knochenwachstum und damit die erstrebte Fusion der beiden Wirbel begünstigt und beschleunigt wird.

Zweckmäßigerweise ist am freien Ende des Seitenauslegers eine Verbreiterung vorgesehen, und zwar jeweils für den Boden bzw. für den Deckel. Mit dieser Verbreiterung wird die Last tragende Fläche im Bereich des freien Endes des Seitenauslegers vergrößert. Die Abstützwirkung wird damit weiter verbessert.

Es hat sich bewährt, den Seitenausleger kürzer zu gestalten, als es der Länge des Tragkörpers entspricht. Ein bevorzugter Wert liegt in einem Bereich vom 0,7 bis 0,9-fachem der Länge des Tragkörpers. Eine für die praktischen Bedürfnisse sehr günstige Geometrie für den aufgespreizten Zustand ergibt sich dann, wenn das Gelenk für den Seitenausleger so positioniert ist, dass im aufgespreizten Zustand der Tragkörper mit dem Seitenausleger ein etwa gleichschenkliges Dreieck bilden. Damit kann eine sichere Abstützung der Endplatten insbesondere in deren randnahen Bereichen, die mit einer härteren kortikalen Schicht versehen sind, erreicht werden. Die Abstützwirkung ist dann wesentlich besser als bei herkömmlichen Implantaten, welche wegen ihrer Kleinheit eher in der Mitte der Endplatten zu positionieren sind, wo die Tragfähigkeit der Wirbel bedeutend geringer ist.

Vorzugsweise ist der Seitenausleger so gestaltet, dass er mindestens um eine Strecke entsprechend dem dreifachen Betrag der Breite des Tragkörpers ausschwenkt. Damit ergibt sich eine Basisbreite für das Implantat, welche eine sichere Abstützung auch mit nur einem einzigen Implantat in einem Wirbelzwischenraum ermöglicht.

Für die praktische Anwendung ist es von Vorteil, wenn eine vorzugsweise anteriore Stirnseite des Tragkörpers abgeschrägt und im Wesentlichen flach ist. Unter anteriorer Stirnseite wird hierbei diejenige verstanden, welche im implantierten Zustand zur frontalen Seite des Patienten weist; dementsprechend ist die posteriore Seite diejenige, welche nach dorsal weist. Indem die Stirnseite abgeflacht ist, wird der Gefahr von Gewebeirritationen an dem den Wirbelkörpern angrenzenden Gewebe, insbesondere den dort verlaufenden großen Gefäßen, verringert. Die Gefahr von Verletzungen als Folge der Implantation ist damit deutlich verringert.

Um eine sichere Betätigung des Zwischenwirbelfusionsimplantats durch denselben Zugang zu ermöglichen, der auch für die Implantation des Implantats vorgesehen ist, ist vorzugsweise ein stirnseitiger Anschluss für die Betätigungseinrichtung vorgesehen, vorzugsweise an einer posterioren Stirnseite. Damit kann die Betätigung durch denselben Zugang erfolgen, ohne dass dazu ein Wechsel des Zugangs oder gar ein Legen eines zusätzlichen Zugangs erforderlich wird.

Die Betätigungseinrichtung ist zweckmäßigerweise selbsthemmend ausgeführt. Damit wird erreicht, dass ohne weiteres Zutun der Seitenausleger in der ausgeschwenkten Position fixiert ist. Eine zweckmäßige Ausführungsform für die Betätigungseinrichtung sieht als Stellglied eine Spindel vor. Dies ermöglicht eine genaue positionstreue Bewegung, da zu jeder Drehbewegung der Spindel ein bestimmtes definiertes, durch die Steigung vorgegebenes Maß der Aufspreizung erreicht wird. Weiter ermöglicht die Spindel ein reversibles Betätigen, so dass gegebenenfalls der Spreizarm auch wieder eingeschwenkt werden kann, falls bei der Implantation zu unvorgesehenen Problemen kommt. Die Spindel bietet weiter den Vorteil, dass sie inhärent selbsthemmend ist.

Wenn es auf die Selbsthemmung nach erfolgter Spreizung nicht ankommen soll, weil beispielsweise der Ausleger durch Verriegelungen sicher in seiner gespreizten Position ohnehin gehaltert ist, kann vorgesehen sein, dass die Spindel entnehmbar ist. Damit kann die Spindel nach Abschluss der Implantation entnommen werden. Dies bietet zum einen den Vorteil, dass nicht unnötig körperfremdes Material verbleibt, und zum anderen bietet dies den Vorteil, dass in dem so geschaffenen Hohlraum im Tragkörper Knochenmaterial, wie beispielsweise Grafts oder Chips, eingebracht werden können. Derart eingebrachtes Knochenmaterial begünstigt das Knochenwachstum und beschleunigt damit die Fusion der benachbarten Wirbelkörper. Vorzugsweise ist eine Öffnung vorgesehen, durch welche bei einer Betätigungseinrichtung ein Zugang zu dem Hohlraum im Tragkörper frei ist. Häufig wird es sich dabei um die Öffnung handeln, durch welche die Betätigungseinrichtung in den Tragkörper geführt ist.

Die Erfindung umfasst ferner ein Set, wobei das Set neben den anspruchsgemäßen Zwischenwirbelfusionsimplantat des Weiteren ein Instrumentarium für das beanspruchte Zwischenwirbelfusionsimplantat umfasst, mit einem Führungsrohr, einem Einsatzstab sowie einem Betätigungsstab. Der Einsatzstab wird in das Führungsrohr eingeschoben und mit dem Zwischenwirbelfusionsimplantat an dessen posterioren Stirnseite verbunden. Zur drehfesten Halterung des Implantats am Führungsrohr weist dieses vorzugsweise eine Zunge auf, die zum Eingriff in eine entsprechende Aussparung des Tragkörpers des Zwischenwirbelimplantats ausgebildet ist. Schließlich wird der Betätigungsstab durch das Führungsrohr mit dem Einsetzstab durchgesteckt. Er dient zum Betätigen der Betätigungseinrichtung. Vorzugsweise trägt er selbst einen Teil der Betätigungseinrichtung, nämlich in Gestalt eines Gewindes an seinem vorderen Ende, wobei das Gewinde als Spindel für die Betätigungseinrichtung fungiert.

Die Erfindung wird nachfolgend unter Bezugnahme auf die beigefügte Zeichnung näher erläutert, in der vorteilhaften Ausführungsbeispiele dargestellt sind. Es zeigen:
- Fig. 1: eine schematische Ansicht für ein erfindungsgemäßes Zwischenwirbelfusionsimplantat im Zwischenwirbelraum zwischen Wirbelkörpern;
- Fig. 2: Darstellungen des Ausführungsbeispiels gemäß Figur 1 in Montagestellung und Wirkstellung;
- Fig. 3: eine perspektivische Darstellung eines ersten Ausführungsbeispiels;
- Fig. 4: Darstellung eines Instrumentariums;
- Fig. 5: eine Detaildarstellung zum Anschluss eines Einsetzinstruments;
- Fig. 6: eine Frontansicht eines zweiten Ausführungsbeispiels;
- Fig. 7: eine perspektivische Darstellung eines zweiten Ausführungsbeispiels;
- Fig. 8: Darstellungen des zweiten Ausführungsbeispiels für einen Zwischenzustand bei der Montage und nach Abschluss der Montage in Wirkstellung; und
- Fig. 9: Darstellungen eines dritten Ausführungsbeispiels in Montage- und Wirkstellung.

Ein in seiner Gesamtheit mit der Bezugsziffer 1 bezeichnetes Zwischenwirbelfusionsimplantat ist zur Implantation in einem Wirbelzwischenraum 91 zwischen zwei unmittelbar benachbarten Wirbelkörpern 9, 9' vorgesehen. Bei physiologisch intakter Wirbelsäule befindet sich zwischen den Wirbeln im Wirbelzwischenraum eine Bandscheibe 90. Sie kann aufgrund von Krankheit oder Verschleiß degeneriert sein, so dass sie zumindest teilweise reseziert werden muss. Um trotz des Verlustes an Bandscheibenmaterial eine ausreichende Abstützung des Wirbelzwischenraums 91 zu erreichen und somit ein Kollabieren der Wirbelsäule zu verhindern, ist das Zwischenwirbelfusionsimplantat 1 in den Wirbelzwischenraum 91 eingesetzt. Es wirkt stützend und erleichtert somit eine Fusion der beiden benachbarten Wirbel 9, 9' auf natürlichem Wege durch Knochenwuchs.

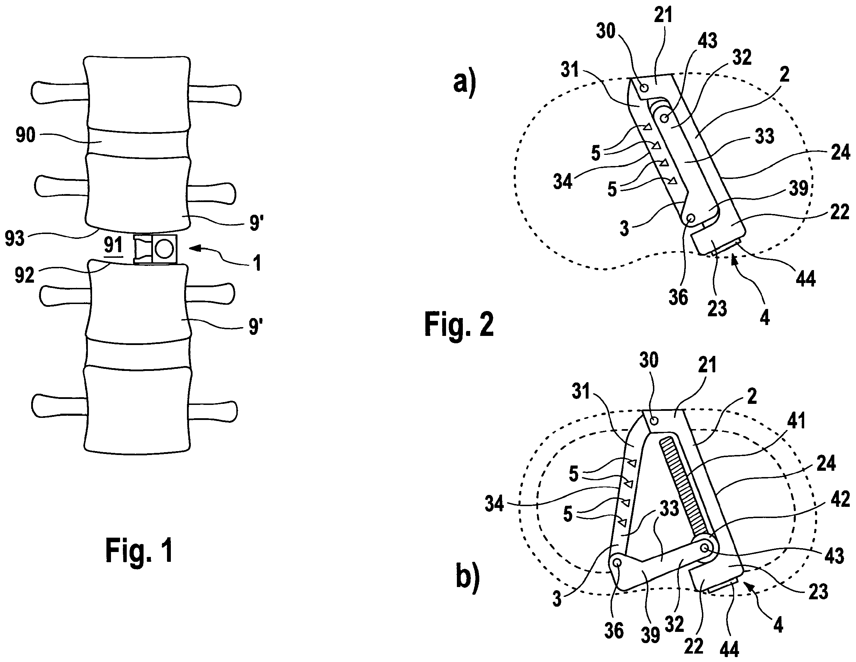

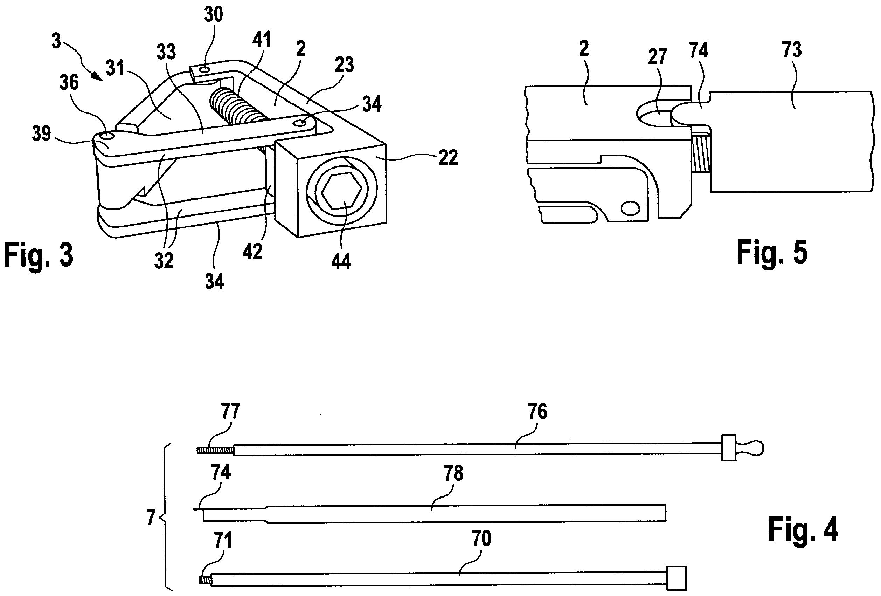

Es wird nun Bezug genommen auf die Darstellung in Figur 2 und 3. Das dort dargestellte erste Ausführungsbeispiel umfasst einen Tragkörper 2 mit einem daran schwenkbar über ein Gelenk 30 angeordneten Seitenausleger 3. Der Seitenausleger ist zweiteilig ausgeführt mit einem Schwenkarm 31, welcher mit einem Ende an dem Gelenk 30 schwenkbeweglich gelagert ist und über ein zweites Gelenk 36 ebenfalls schwenkbeweglich mit einem Spreizarm 32 verbunden ist. Es ergibt sich damit eine Konstruktion, die kniehebelartig ausschwenken kann (vgl. Fig. 2b).

Ferner vorgesehen ist eine Betätigungseinrichtung 4, welche in dem dargestellten Ausführungsbeispiel eine integrierte Betätigungsspindel 41 sowie ein Gleitstück 42 umfasst. Das Gleitstück 42 ist über einen Haltestift 43 schwenkbeweglich an dem freien Ende des Spreizarms 32 angeordnet. Das Gleitstück 42 weist in seiner Mitte eine Durchgangsbohrung mit einem Innengewinde auf. Durch dieses ist die Spindel 41 der Betätigungseinrichtung geführt, welche mit ihrem Kopf 44 in einer posterioren Stirnwand 22 des Tragkörpers 2 gelagert ist. Wird die Spindel 41 durch Verdrehen des Spindelkopfs gedreht, so bewegt sich das Gleitstück 42 ausgehend von einer Montageposition (s. Fig. 2a) am anterioren Ende der Spindel nach posterior, wobei der Schwenkarm 31 und der Spreizarm 32 kniegelenkartig nach lateral ausschwenken.

Der Tragkörper 2 weist an seiner anterioren, vorderen Stirnseite 21 eine flache Abschrägung auf. Sie weist einen Winkel von etwa 20° gegenüber einer Orthogonalen zu einer von der Spindel 41 gebildeten Längsachse des Tragkörpers 2 auf. Mit dieser Abschrägung wird eine flache, nicht hervorstehende Gestaltung der vorderen Stirnseite erreicht. Die Gefahr von Irritationen von vor dem Wirbel liegendem Gewebe ist damit minimiert.

Der Tragkörper 2 weist an seiner Oberseite eine Deckfläche 23 und an seiner Unterseite entsprechend eine Bodenfläche 24 auf. Sie dienen zur Anlage an die entsprechenden Endplatten 92, 93 der beiden benachbarten Wirbelkörper 9, 9'. Mit ihnen auf einer Höhe fluchtend befindet sich der Deckel 33 bzw. der Boden 34 des Seitenauslegers 3. Damit ist erreicht, dass eine Unterstützung am ausgeschwenkten Seitenausleger niveaugleich mit derjenigen des Tragkörpers 2 erfolgt. In der Montagestellung liegt der Deckel 33 bündig an der Deckfläche 23 des Tragkörpers an, dasselbe gilt entsprechend für den Boden 34 in Bezug auf die Bodenfläche 24.

Auf dem Deckel 33 bzw. an dem Boden 34 (dort nicht dargestellt) ist eine Verzahnung 5 angeordnet. Die Zähne sind so ausgerichtet, dass sie bei dem Ausschwenken des Seitenauslegers 3 Knochenmaterial von der zugeordneten Endplatte 92, 93 abtragen und damit eine Anfrischung des Knochens in diesem Bereich durchführen.

Zur Implantation ist ein Instrumentarium 7 vorgesehen, welches in Figur 4 dargestellt ist. Es umfasst einen Einsetzstab 70, ein Führungsrohr 73 sowie einen Betätigungsstab 76. Der Einsatzstab wird durch das Führungsrohr 73 geschoben und mit seinem vorderen Ende an dem Tragkörper 2 des Zwischenwirbelfusionsimplantats 1 befestigt. Der so geschaffene Verbund kann durch einen minimal invasiven Zugang, der beispielsweise im Rahmen des PLIF-Verfahrens (Posterior Lumbar Intervertebral Fusion) geschaffen ist, in dem Zwischenwirbelraum 91 eingebracht werden. Es nimmt dann dort die in Figur 2a dargestellte Position ein. In einem nächsten Schritt erfolgt ein Aufspreizen durch die Betätigungseinrichtung. In dem dargestellten Ausführungsbeispiel gemäß Figur 2 und 3 wird dazu ein geeigneter Schraubantrieb in den Schraubkopf 44 der Betätigungsspindel 41 eingesetzt, und damit durch Verdrehen der Spindel 41 der Seitenausleger 3 ausgeschwenkt. Das geschieht in der Weise, indem durch das Verdrehen der Spindel 41 das Gleitstück 42 nach posterior, also zum Schraubkopf 44 hin, gezogen wird, und somit der dort angelegte Spreizarm 32 den Seitenarm 31 ausschwenkt. Schließlich wird die in Figur 2b dargestellte Montageposition erreicht.

Man erkennt in Figur 2b, dass insbesondere die mit Strichlinie kenntlich gemachte harte kortikale Umrandung des Wirbelkörpers in einer für die Kraftübertragung günstigen Weise auf breitflächigen Anteilen an der Deckel- bzw. Bodenfläche aufliegt, nämlich im Bereich der posterioren Stirnseite und der anterioren Stirnseite des Tragkörpers 2 sowie an der an dem Seitenausleger 3 vorgesehenen Verbreiterung 39. Dank dieser breiten Gestaltung kann auch mit einem verhältnismäßig kleinen Implantat eine hervorragende Kraftübertragung erreicht werden.

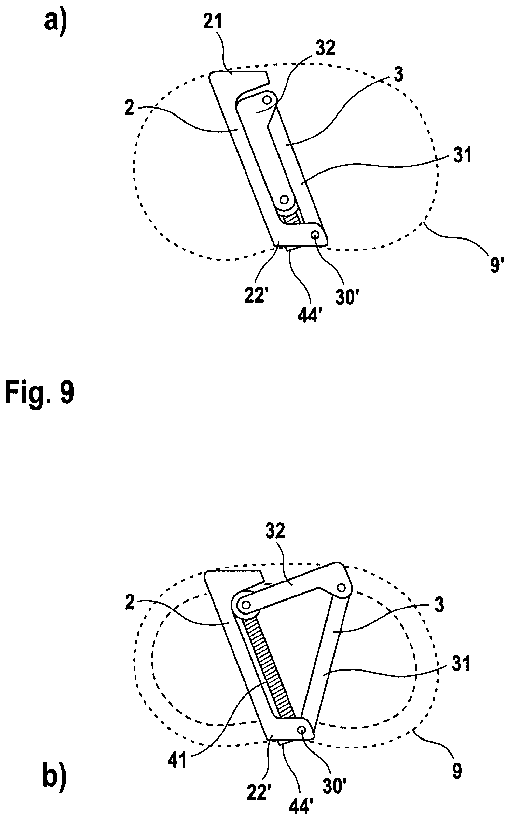

Bei dem in Fig. 9 dargestellten dritten Ausführungsbeispiel handelt es sich um eine Variante des ersten Ausführungsbeispiels. Sie unterscheidet sich im Wesentlichen dadurch, dass der Seitenausleger 3 am posterioren Ende mit einem Gelenk 30' angelenkt ist und nicht am anterioren Ende wie bei der in Fig. 1 bis 3 dargestellten ersten Ausführungsform. Das Zwischenwirbelfusionsimplantat gemäß dem dritten Ausführungsbeispiel spreizt sich daher entsprechend umgekehrt auf, also nach anterior. Damit befindet sich ein Schraubkopf 44' für die Betätigungsspindel 41 an derselben Seite wie das Gelenk 30', welches entsprechend seitwärts versetzt ist, um hier ausreichend Bauraum zu schaffen. Weiter ist bei dieser Ausführungsform auch die posteriore Stirnseite 23' abgeschrägt, um einen möglichst glatten Abschluss ohne Irritationsgefahr von umliegendem Gewebe zu erreichen. Im Übrigen haben dieselben Teile dieselben Funktionen wie bei dem zuvor beschriebenen ersten Ausführungsbeispiel. Sie sind auch mit denselben Bezugsziffern versehen. Es wird insoweit auf die vorstehende Beschreibung verwiesen.

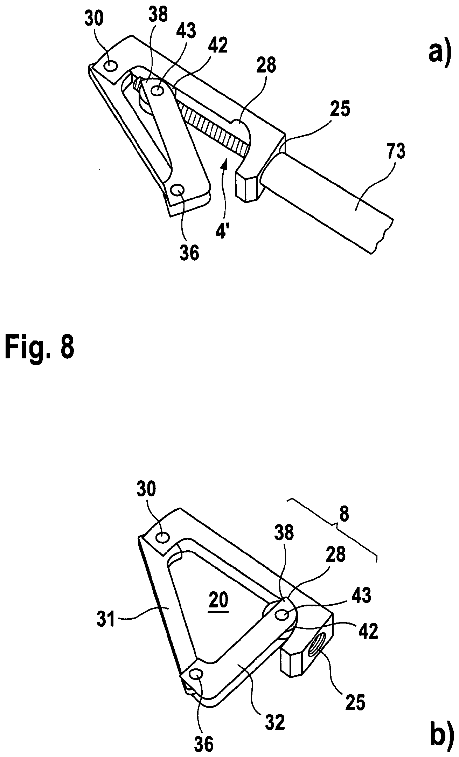

Als zweites Ausführungsbeispiel wird unter Bezugnahme auf Figur 5-8 erläutert. Es ist im Wesentlichen gleichartig zu dem ersten Ausführungsbeispiel aufgebaut, wobei gleiche bzw. gleichartige Elemente dieselben Bezugsziffern tragen. Es unterscheidet sich im Hinblick auf die Betätigungseinrichtung 4'. So sieht die Betätigungseinrichtung 4' ein Gleitstück 42 vor, über das in derselben Weise wie bei dem ersten Ausführungsbeispiel eine Spreizung des Seitenauslegers 3 erreicht wird. Jedoch umfasst die Betätigungseinrichtung 4' keine eigene Spindel, sondern lediglich eine entsprechende Spindellagerung in einer vergrößerten Öffnung 25 an der posterioren Stirnseite 22 des Tragkörpers. Diese vergrößerte Öffnung 25 kann mit einem Innengewinde versehen sein. Ihr Durchmesser ist etwa doppelt so groß wie das durch das Gelenkstück führende Durchgangsloch mit dem Gewinde.

Ferner ist an dem Tragkörper 2 an der Bodenseite 24 eine von der Stirnseite 22 sich aus erstreckende U-förmige Ausnehmung 27 vorgesehen. Sie dient zur Aufnahme einer Fixierungszunge des Instrumentariums, wie später noch erläutert wird.

Ferner sind in dem Bereich des Tragkörpers, in dem das Gleitstück 42 in der Wirkstellung positioniert ist, Ausnehmungen 28 an Deck- und Bodenfläche 23, 24 angeordnet. Sie sind so ausgeformt, dass sie das gleitstückseitige Ende des Spreizarms 32 formschlüssig aufnehmen. Auf diese Weise ist der Spreizarm in anterior-posteriorer Richtung verriegelt, wodurch auch der Schwenkarm 31 in seiner ausgeschwenkten Position verriegelt ist. Zur Gewährleistung einer sicheren, formschlüssigen Verriegelung ist das gelenkstückseitige Ende des Spreizarms 32 mit Rastkanten 38 versehen. Die Ausnehmungen 28 und 38 bilden damit gemeinsam eine Verriegelungseinrichtung 8.

Dank der von der Betätigungseinrichtung 4 unabhängigen Verriegelungseinrichtung 8 ist es nach dem Ausschwenken des Seitenauslegers 3 nicht mehr erforderlich, dass eine Spindel 41 im Implantat verbleibt. Sie kann daher entnommen werden. Damit wird die zur Aufnahme des Spindelkopfs 44 vorgesehene Öffnung 25 frei und kann als Zugangsöffnung zu einem Innenraum 20 des Tragkörpers 2 fungieren. Damit können nach dem Einsetzen des Implantats und Spreizen in die Wirkstellung durch den minimal invasiven Zugang Grafts oder Chips mit Knochenmaterial eingebracht werden, um so die Fusion der beiden angrenzenden Wirbelkörper 9, 9' zu begünstigen.

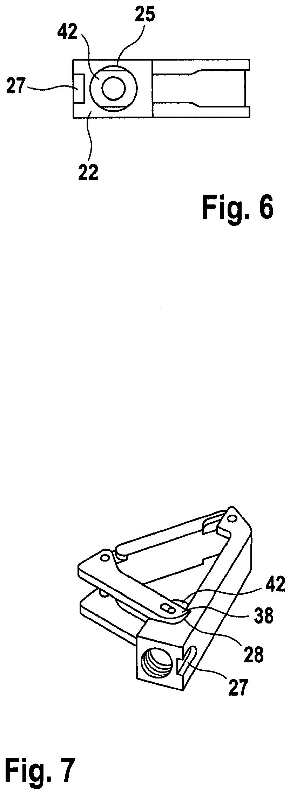

Nachfolgend wird unter Bezugnahme auf das Instrumentarium, wie in Figur 4 dargestellt, die Implantation beschrieben. Das Führungsrohr 73 weist an seinem vorderen Ende eine Zunge 74 auf, welche formschlüssig in die Ausnehmung 27 in den Tragkörper 2 eingreift und diesen damit drehfest am Führungsrohr 73 fixiert. Der Einsetzstab 70 wird durch das Führungsrohr 73 geschoben und mit seinem Gewinde 71 am vorderen Ende in das Innengewinde der Öffnung 25 eingeschraubt. Damit ist das Implantat an dem Führungsrohr 73 an dem Einsatzstab 70 fest gehaltert (s. Fig. 5). Im nächsten Schritt wird der Betätigungsstab 76 durch den Einsatzstab 70 geführt, der zu diesem Zweck hohl gebohrt ist. Der Betätigungsstab 76 greift mit seinem Gewinde 77 in das Gleitstück 42. Das Implantat 1' ist damit in seiner Montageposition an dem Instrumentarium aufgenommen, wie in Figur 6 dargestellt. Es kann dann durch den minimal invasiven Zugang in den Wirbelzwischenraum 91 eingebracht werden. Hiernach wird durch Verdrehen des Betätigungsstabs 76, der als Spindel der für die Betätigungseinrichtung 4 fungiert, das Gleitstück 42 nach posterior bewegt, wodurch der Spreizarm 32 nach außen bewegt wird und entsprechend der Schwenkarm 31 seitlich ausgeschwenkt wird. Die Montageposition mit voll ausgeschwenktem Schwenkarm 31 ist erreicht, wenn die Rastnasen 38 der Verriegelungseinrichtung 8 in die Ausnehmung 28 eingreifen. Damit ist das Implantat verriegelt. Der Betätigungsstab 76 kann dann entnommen werden, und ebenso kann das Führungsrohr 73 mit dem Einsatzstift 70 entfernt werden.

## Patentansprüche

1. Zwischenwirbelfusionsimplantat zur Fusion zweier benachbarter Wirbel (9, 9') umfassend
- einen verstellbaren Tragkörper (2), dessen Bodenund Deckfläche (23, 24) zur Anlage an Endplatten (92, 93) der benachbarten Wirbel (9, 9') ausgebildet sind,
- ein um ein Gelenk (30) lateral schwenkbarer Seitenausleger (3), dessen Boden (34) und Deckel (33) flächenmäßig ausgebildet sind, und
- eine Betätigungseinrichtung (4) zum Ausschwenken des Seitenauslegers (3) in eine vom Tragkörper (2) abgespreizte Position, wobei
- der Seitenausleger (3) als ein Kniehebelspreizer mit einem Schwenkarm (31) und einem Spreizarm (32) ausgeführt ist und der Spreizarm (32) in der Wirkstellung am Tragkörper (2) verriegelt wird,
**dadurch gekennzeichnet, dass**
am Spreizarm (32) ein Gleitstück (42) befestigt ist, welches von der Betätigungseinrichtung (4) beim Spreizen verschoben wird und bei Erreichen der Wirkstellung vorzugsweise in Ausnehmungen (28) verrastet.

2. Zwischenwirbelfusionsimplantat nach Anspruch 1,
**dadurch gekennzeichnet, dass** in einer Montagestellung der Seitenausleger (3) in den Tragkörper (2) eingezogen ist.

3. Zwischenwirbelfusionsimplantat nach Anspruch 2,
**dadurch gekennzeichnet, dass** der Boden (34) und/oder Deckel (33) des Seitenauslegers (3) in der Montagestellung bündig anliegen an den Boden- und/oder Deckflächen (23, 24) des Tragkörpers (2).

4. Zwischenwirbelfusionsimplantat nach einem der vorangehenden Ansprüche, **dadurch gekennzeichnet, dass** der Boden (34) und/oder Deckel (33) des Seitenauslegers (3) fluchtet mit der Boden- und/oder Deckfläche (23, 24) des Tragkörpers (2).

5. Zwischenwirbelfusionsimplantat nach Anspruch 4,
**dadurch gekennzeichnet, dass** an dem Boden (34) und/oder Deckel (33) eine Schneidverzahnung (5) angeordnet ist.

6. Zwischenwirbelfusionsimplantat nach einem der vorangehenden Ansprüche, **dadurch gekennzeichnet, dass** am freien Ende des Seitenauslegers (3) eine Verbreiterung (39) des Bodens (34) und/oder Deckels (33) vorgesehen ist.

7. Zwischenwirbelfusionsimplantat nach einem der vorangehenden Ansprüche, **dadurch gekennzeichnet, dass** der Seitenausleger (3) eine Länge von etwa 0,7 bis 0,9-fachem der Länge des Tragkörpers (2) aufweist.

8. Zwischenwirbelfusionsimplantat nach Anspruch 7,
**dadurch gekennzeichnet, dass** das Gelenk (30) für den Seitenausleger (3) so positioniert ist, dass der Tragkörper (2) und der ausgespreizte Seitenausleger (3) ein etwa gleichschenkliges Dreieck bilden.

9. Zwischenwirbelfusionsimplantat nach einem der vorangehenden Ansprüche, **dadurch gekennzeichnet, dass** der Seitenausleger (3) mindestens um eine Strecke entsprechend dem dreifachen Betrag der Breite des Tragkörpers (2) ausschwenkt.

10. Zwischenwirbelfusionsimplantat nach einem der vorangehenden Ansprüche, **dadurch gekennzeichnet, dass** eine, vorzugsweise anteriore, Stirnseite (21) des Tragkörpers (2) abgeschrägt und im Wesentlichen flach ist.

11. Zwischenwirbelfusionsimplantat nach einem der vorangehenden Ansprüche, **dadurch gekennzeichnet, dass** ein Anschluss (44) für die Betätigungseinrichtung (4) an einer vorzugsweise posterioren, Stirnseite (22) vorgesehen ist.

12. Zwischenwirbelfusionsimplantat nach einem der vorangehenden Ansprüche, **dadurch gekennzeichnet, dass** die Betätigungseinrichtung (4) selbsthemmend ist.

13. Zwischenwirbelfusionsimplantat nach einem der vorangehenden Ansprüche, **dadurch gekennzeichnet, dass** die Betätigungseinrichtung (4) als Stellglied eine Spindel (41) aufweist.

14. Zwischenwirbelfusionsimplantat nach Anspruch 13,
**dadurch gekennzeichnet, dass** die Spindel (41) entnehmbar ist.

15. Zwischenwirbelfusionsimplantat nach einem der vorangehenden Ansprüche, **dadurch gekennzeichnet, dass** eine Öffnung (25) vorgesehen ist, durch welche bei entnommener Betätigungseinrichtung (4) ein Zugang zu einem Hohlraum (20) im Tragkörper (2) frei ist.

16. Zwischenwirbelfusionsimplantat nach einem der vorangehenden Ansprüche, **dadurch gekennzeichnet, dass** am Tragkörper (2) eine Befestigung für einen Einsatzstab (70) angeordnet ist, vorzugsweise als eine Öffnung (25) mit Innengewinde.

17. Zwischenwirbelfusionsimplantat nach einem der vorangehenden Ansprüche, **dadurch gekennzeichnet, dass** am Tragkörper (2) eine Kupplung (27) für eine Drehmomentstütze angeordnet ist.

18. Zwischenwirbelfusionsimplantat nach einem der vorangehenden Ansprüche, **dadurch gekennzeichnet, dass** am Tragkörper (2) eine Verriegelungseinrichtung (8) für den Seitenausleger (3) vorgesehen ist.

19. Set umfassend
- ein Zwischenwirbelfusionsimplantat nach einem der vorangehenden Ansprüche und
- ein Instrumentarium für ein Zwischenwirbelfusionsimplantat nach einem der vorangehenden Ansprüche, wobei das Instrumentarium (7) ein Führungsrohr (73), einen Einsatzstab (70) sowie einen Betätigungsstab (76) umfasst.

20. Set nach Anspruch 19, **dadurch gekennzeichnet, dass** am Führungsrohr (73) am vorderen Ende eine Zunge (74) zum Eingriff in den Tragkörper (2) des Zwischenwirbelfusionsimplantats angeordnet ist.

21. Set nach Anspruch 19 oder 20, **dadurch gekennzeichnet, dass** der Betätigungsstab (76) ein Gewinde (77) an seinem vorderen Ende aufweist, welches als Spindel für die Betätigungseinrichtung (4) fungiert.

## Claims

1. Intervertebral fusion implant for fusing two adjacent vertebrae (9, 9'), comprising
- an adjustable support body (2), the base surface and cover surface (23, 24) of which are configured to bear on end plates (92, 93) of the adjacent vertebrae (9, 9'),
- a side bracket (3), which can be pivoted laterally about a hinge (30) and the base (34) and cover (33) of which have a planar design, and
- an actuation means (4) for pivoting out the side bracket (3) into a position spread from the support body (2), wherein
- the side bracket (3) is configured as a toggle expander with a pivot arm (31) and a spreader arm (32) and the spreader arm (32) is locked on the support body (2) in the working position, **characterized in that** a sliding piece (42) is attached to the spreader arm (32) and displaced by the actuation means (4) during the spreading and it preferably latches into recesses (28) when the working position is reached.

2. Intervertebral fusion implant according to Claim 1, **characterized in that** the side bracket (3) is retracted into the support body (2) in an assembly position.

3. Intervertebral fusion implant according to Claim 2, **characterized in that** the base (34) and/or cover (33) of the side bracket (3) lie flush against the base and/or cover surfaces (23, 24) of the support body (2) in the assembly position.

4. Intervertebral fusion implant according to one of the preceding claims, **characterized in that** the base (34) and/or cover (33) of the side bracket (3) is aligned with the base and/or cover surface (23, 24) of the support body (2).

5. Intervertebral fusion implant according to Claim 4, **characterized in that** cutting teeth (5) are arranged on the base (34) and/or cover (33).

6. Intervertebral fusion implant according to one of the preceding claims, **characterized in that** a widening (39) of the base (34) and/or cover (33) is provided at the free end of the side bracket (3).

7. Intervertebral fusion implant according to one of the preceding claims, **characterized in that** the side bracket (3) has a length of approximately 0.7 to 0.9 times the length of the support body (2).

8. Intervertebral fusion implant according to Claim 7, **characterized in that** the hinge (30) for the side bracket (3) is positioned in such a way that the support body (2) and the spread side bracket (3) form an approximate isosceles triangle.

9. Intervertebral fusion implant according to one of the preceding claims, **characterized in that** the side bracket (3) pivots out at least by a travel corresponding to three times the value of the width of the support body (2).

10. Intervertebral fusion implant according to one of the preceding claims, **characterized in that** an end face (21), preferably an anterior end face, of the support body (2) is beveled and substantially flat.

11. Intervertebral fusion implant according to one of the preceding claims, **characterized in that** provision is made on an end face (22), preferably on a posterior end face, for a connector (44) for the actuation means (4).

12. Intervertebral fusion implant according to one of the preceding claims, **characterized in that** the actuation means (4) is self-retaining.

13. Intervertebral fusion implant according to one of the preceding claims, **characterized in that** the actuation means (4) has a spindle (41) as actuator.

14. Intervertebral fusion implant according to Claim 13, **characterized in that** the spindle (41) is removable.

15. Intervertebral fusion implant according to one of the preceding claims, **characterized in that** provision is made for an opening (25) through which, when the actuation means (4) is removed, there is a clear access to a cavity (20) in the support body (2) .

16. Intervertebral fusion implant according to one of the preceding claims, **characterized in that** an attachment for an insertion rod (70) is arranged on the support body (2), preferably as an opening (25) with a female thread.

17. Intervertebral fusion implant according to one of the preceding claims, **characterized in that** a coupling (27) for a torque support is arranged on the support body (2).

18. Intervertebral fusion implant according to one of the preceding claims, **characterized in that** a locking means (8) for the side bracket (3) is provided on the support body (2).

19. Set comprising
- an intervertebral fusion implant according to one of the preceding claims and
- an instrument set for an intervertebral fusion implant according to one of the preceding claims, wherein the instrument set (7) comprises a guide tube (73), an insertion rod (70) and an actuation rod (76).

20. Set according to Claim 19, **characterized in that** a tongue (74) for engagement into the support body (2) of the intervertebral fusion implant is arranged at the front end of the guide tube (73).

21. Set according to Claim 19 or 20, **characterized in that** the actuation rod (76) has a thread (77) at the front end thereof, which acts as a spindle for the actuation means (4).

## Revendications

1. Implant de fusion intervertébral pour la fusion de deux vertèbres adjacentes (9, 9'), comprenant :
- un corps de support réglable (2), dont la surface inférieure et la surface supérieure (23, 24) sont réalisées de manière à s'appliquer contre des plateaux d'extrémité (92, 93) des vertèbres adjacentes (9, 9'),
- un bras latéral (3) pouvant pivoter latéralement autour d'une articulation (30), dont la partie inférieure (34) et la partie supérieure (33) sont réalisées sous forme plane et
- un dispositif d'actionnement (4) pour sortir par pivotement le bras latéral (3) dans une position écartée du corps de support (2),
- le bras latéral (3) étant réalisé sous forme d'écarteur à levier basculant avec un bras pivotant (31) et un bras d'écartement (32) et le bras d'écartement (32) étant verrouillé dans la position fonctionnelle sur le corps de support (2),
**caractérisé en ce**
**qu'**un coulisseau (42) est fixé au bras d'écartement (32), lequel est déplacé par le dispositif d'actionnement (4) lors de l'écartement et s'encliquète de préférence dans des évidements (28) une fois que la position fonctionnelle est atteinte.

2. Implant de fusion intervertébral selon la revendication 1,
**caractérisé en ce que**
dans une position de montage, le bras latéral (3) est enfoncé dans le corps de support (2).

3. Implant de fusion intervertébral selon la revendication 2,
**caractérisé en ce que** la partie inférieure (34) et/ou la partie supérieure (33) du bras latéral (3) s'appliquent en affleurement dans la position de montage contre les surfaces inférieure et/ou supérieure (23, 24) du corps de support (2).

4. Implant de fusion intervertébral selon l'une quelconque des revendications précédentes, **caractérisé en ce que** la partie inférieure (34) et/ou la partie supérieure (33) du bras latéral (3) sont en affleurement avec la surface inférieure et/ou supérieure (23, 24) du corps de support (2).

5. Implant de fusion intervertébral selon la revendication 4,
**caractérisé en ce qu'**une denture de découpage (5) est disposée au niveau de la partie inférieure (34) et/ou de la partie supérieure (33).

6. Implant de fusion intervertébral selon l'une quelconque des revendications précédentes, **caractérisé en ce qu'**un élargissement (39) de la partie inférieure (34) et/ou de la partie supérieure (33) est prévu au niveau de l'extrémité libre du bras latéral (3).

7. Implant de fusion intervertébral selon l'une quelconque des revendications précédentes, **caractérisé en ce que** le bras latéral (3) présente une longueur comprise entre 0,7 et 0,9 fois la longueur du corps de support (2).

8. Implant de fusion intervertébral selon la revendication 7, **caractérisé en ce que** l'articulation (30) pour le bras latéral (3) est positionnée de telle sorte que le corps de support (2) et le bras latéral écarté (3) forment un triangle approximativement isocèle.

9. Implant de fusion intervertébral selon l'une quelconque des revendications précédentes, **caractérisé en ce que** le bras latéral (3) pivote vers l'extérieur sur au moins une section correspondant à trois fois la valeur de la largeur du corps de support (2).

10. Implant de fusion intervertébral selon l'une quelconque des revendications précédentes, **caractérisé en ce qu'**un côté frontal (21), de préférence antérieur, du corps de support (2) est biseauté et est essentiellement plat.

11. Implant de fusion intervertébral selon l'une quelconque des revendications précédentes, **caractérisé en ce qu'**un raccord (44) pour le dispositif d'actionnement (4) est prévu au niveau d'un côté frontal (22), de préférence postérieur.

12. Implant de fusion intervertébral selon l'une quelconque des revendications précédentes, **caractérisé en ce que** le dispositif d'actionnement (4) est auto-bloquant.

13. Implant de fusion intervertébral selon l'une quelconque des revendications précédentes, **caractérisé en ce que** le dispositif d'actionnement (4) présente une broche (41) en tant qu'actionneur.

14. Implant de fusion intervertébral selon la revendication 13,
**caractérisé en ce que** la broche (41) peut être enlevée.

15. Implant de fusion intervertébral selon l'une quelconque des revendications précédentes, **caractérisé en ce qu'**il est prévu une ouverture (25) à travers laquelle, lorsque le dispositif d'actionnement (4) est enlevé, un accès à une cavité (20) dans le corps de support (2) est possible.

16. Implant de fusion intervertébral selon l'une quelconque des revendications précédentes, **caractérisé en ce qu'**une fixation pour une barre d'insertion (70) est disposée sur le corps de support (2), de préférence sous forme d'ouverture (25) avec un filetage intérieur.

17. Implant de fusion intervertébral selon l'une quelconque des revendications précédentes, **caractérisé en ce qu'**un accouplement (27) pour un support de couple est disposé sur le corps de support (2).

18. Implant de fusion intervertébral selon l'une quelconque des revendications précédentes, **caractérisé en ce qu'**un dispositif de verrouillage (8) pour le bras latéral (3) est prévu sur le corps de support (2).

19. Ensemble, comprenant
- un implant de fusion intervertébral selon l'une quelconque des revendications précédentes, et
- un kit d'instruments pour un implant de fusion intervertébral selon l'une quelconque des revendications précédentes, le kit d'instruments (7) comprenant un tube de guidage (73), une barre d'insertion (70) ainsi qu'une barre d'actionnement (76).

20. Ensemble selon la revendication 19, **caractérisé en ce qu'**une langue (74) destinée à s'engager dans le corps de support (2) de l'implant de fusion intervertébral est disposée à l'extrémité avant sur le tube de guidage (73).

21. Ensemble selon la revendication 19 ou 20, **caractérisé en ce que** la barre d'actionnement (76) présente un filetage (77) à son extrémité avant, lequel sert de broche pour le dispositif d'actionnement (4).
